Europäisches Patentamt

European Patent Office

Office européen des brevets

(11) Numéro de publication: **0 277 267
B1**

# (12) FASCICULE DE BREVET EUROPEEN

(45) Date de publication du fascicule du brevet:
31.10.90

(51) Int. Cl.⁵: **C07D 209/52**

(21) Numéro de dépôt: **87108477.8**

(22) Date de dépôt: **12.06.87**

(54) **Procédé de synthèse du N-amino aza-3 bicyclo [3,3,0] octane.**

(30) Priorité: **04.02.87 FR 8701334**

(43) Date de publication de la demande:
**10.08.88 Bulletin 88/32**

(45) Mention de la délivrance du brevet:
**31.10.90 Bulletin 90/44**

(84) Etats contractants désignés:
**AT BE CH DE FR GB IT LI LU NL SE**

(56) Documents cités:
**FR-A- 2 223 017
FR-A- 2 540 110
OA-A- 4 735**

(73) Titulaire: **Oril S.A., 13 rue Auguste Desgenétais,
F-76210 Bolbec(FR)**

(72) Inventeur: **Cohen, Adad Roger, 1, rue du Tonkin,
F-69100 Villeurbanne(FR)**
Inventeur: **Cohen, Armand, 12 rue du Président Coty,
F-76210 Bolbec(FR)**
Inventeur: **Delalu, Henri, 35 rue Bichat, F-69002 Lyon(FR)**
Inventeur: **Marchand, Alain, 5 avenue Jean Cagne,
F-69200 Venissieux(FR)**
Inventeur: **Mauge, Robert, 7 rue Edouard Herriot,
F-76600 Le Havre(FR)**

(74) Mandataire: **TER MEER - MÜLLER - STEINMEISTER &
PARTNER, Mauerkircherstrasse 45,
D-8000 München 80(DE)**

## Description

La présente invention concerne un nouveau procédé de synthèse du N-amino aza-3 bicyclo [3,3,0] octane.

Le N-amino aza-3 bicyclo [3,3,0] octane ou N-amino cyclopenta-[c]-pyrrole octahydro, est un composé utilisé très fréquemment comme intermédiaire de synthèse des médicaments (Brevet GB N°1.153.982).

A l'heure actuelle, la seule méthode décrite dans la littérature pour la préparation de ce composé est le procédé de Wright J.B. et Willette R.E. (J.Med. and Pharm. Chem. 1962,5,819) qui consiste en une nitrosation de l'aza-3 bicyclo [3,3,0] octane et ensuite en une réduction du dérivé N-nitrosé obtenu. Cette synthèse conduit à d'assez bond rendements. Toutefois, elle nécessite deux étapes distinctes et le produit issu de la première étape doit être manipulé avec beaucoup de précaution à cause de sa toxicité potentielle, ce qui pose industriellement des problèmes de mise en exploitation.

D'autre part, il est reconnu que, pour la préparation des différentes hydrazines, on fait souvent appel à la réaction dite de "Raschig", qui consiste à synthétiser la monochloramine par réaction de l'ammoniac sur une solution d'hypochlorite de sodium et ensuite à faire réagir la monochloramine formée sur une amine pour obtenir l'hydrazine correspondante. Ce procédé est assez difficile à mettre en œuvre parce qu'il nécessite deux étapes distinctes, la première réalisée à froid pour la synthèse de la monochloramine et la deuxième réalisée à chaud, pendant laquelle est effectuée la synthèse proprement dite de l'hydrazine. Par ailleurs, la monochloramine doit se trouver en présence d'un excès suffisant d'amine dans les solutions intermédiaires de manière à éviter des réactions secondaires de dégradation, et par la suite le procédé exige toujours des quantités très importantes de solutions à traiter.

Le brevet français, demande N° 76.34692, décrit un procédé de synthèse de N,N-diméthylhydrazine réalisé en continu à partir d'ammoniac, d'hypochlorite de sodium et de la diméthylamine en milieu aqueux.

Cependant, ce procédé ne peut pas être appliqué pour la préparation de toutes alkylhydrazines et surtout pas pour la préparation du N-amino aza-3 bicyclo [3,3,0] octane, compte tenu de ce que la matière première nécessaire pour sa synthèse, l'aza-3 bicyclo [3,3,0] octane a des propriétés physicochimiques particulieres, tres différentes de celles de la diméthylamine. D'une part, cette amine bicyclique est soluble dans des solutions aqueuses alcalines a des concentrations moyennes et surtout à froid. D'autre part, elle peut reagir avec la monochloramine seulement à des températures élevées, auxquelles les mélanges eau et aza-3 bicyclo [3,3,0] octane subissent une démixtion.

La demanderesse a maintenant découvert un nouveau procédé de synthèse du N-amino aza-3 bicyclo [3,3,0] octane. Ce procédé, mis en oeuvre en continu, repose en partie sur une transposition du procédé Raschig, qui consiste à préparer la chloramine par action de l'hypochlorite de sodium sur l'ammoniac à basse température, et ensuite, ce qui constitue d'ailleurs sa grande originalité, à faire agir la chloramine ainsi produite sur l'aza-3 bicyclo [3,3,0] octane en milieu biphasique, puis à extraire l'hydrazine formée et à récupérer et recycler l'amine de départ sous la forme d'une solution aqueuse directement sans aucune étape supplémentaire.

La présente invention a plus particulièrement pour objet un procédé de synthèse en continu du N-amino aza-3 bicyclo [3,3,0] octane, caractérisé en ce que l'on fait réagir une solution d'hydroxyde d'ammonium et de chlorure d'ammonium avec une solution aqueuse d'hypochlorite de sodium à une température comprise entre-15°C et-7°C en milieu alcalin,

- et ensuite, que l'on fait réagir la monochloramine ainsi formée avec l'aza-3 bicyclo [3,3,0] octane, en milieu biphasique dans un réacteur approprié muni d'un agitateur coaxial à ailettes, à une température comprise entre 30°C et 90°C et en milieu alcalin,

- puis que l'on sépare du milieu réactionnel l'ammoniac et ensuite l'aza-3 bicyclo [3,3,0] octane qui n'a pas réagi par distillation, pour le recycler,

- puis que l'on isole par démixtion une solution concentrée de N-amino aza-3 bicyclo [3,3,0] octane par addition d'hydroxyde de sodium au milieu réactionnel, et que l'on purifie l'hydrazine ainsi obtenue, si on le désire, par distillation.

Pour former la monochloramine, une solution aqueuse d'hypochlorite de sodium est mélangée sous agitation avec une solution d'hydroxyde d'ammonium et de chlorure d'ammonium. La réaction se réalise en milieu alcalin d'un pH de 9,2 à 10 en présence d'un excès d'hydroxyde d'ammonium et de chlorure d'ammonium. Le rapport des concentrations molaires hydroxyde d'ammonium et chlorure d'ammonium sur hypochlorite de sodium est environ 2,5 à 3 et le rapport des concentrations molaires chlorure d'ammonium sur hydroxyde d'ammonium est d'environ 0.50 à 0.80.

La réaction de la monochloramine avec l'aza-3 bicyclo [3,3,0] octane s'effectue en présence d'une solution aqueuse d'hydroxyde de sodium à chaud. Le rapport des concentrations molaires d'aza-3 bicyclo [3,3,0] octane sur monochloramine doit être supérieur à 4 et inférieur à 8. Le temps de réaction est variable et dépend de la température à laquelle s'effectue la réaction et du rapport des concentrations des réactifs et il est de l'ordre de 20 à 40 secondes.

Après formation de N-amino aza-3 bicyclo [3,3,0] octane et refroidissement, la solution réactionnelle subit un dégazage pour éliminer l'ammoniac et l'amino-3 bicyclo [3,3,0] octane qui n'a pas réagi est séparé du milieu réactionnel par simple distillation sous pression atmosphérique et à une température environ 90° à 100°C. Sous ces conditions, l'amine est obtenue sous forme d'une solution aqueuse de concentration 30% en aza-3 bicyclo [3,3,0] octane. Cette solution est recyclée immédiatement.

La solution réactionnelle contenant l'hydrazine est ensuite traitée par la soude. Cette opération, conduite en deux étapes, permet la séparation de l'N-amino aza-3 bicyclo [3,3,0] octane dans une phase organique de concentration 92% en hydrazine. La solution concentrée ainsi obtenue peut être utilisée directement ou distillée sous pression réduite.

Le procédé de la présente invention permet donc non seulement la synthèse du N-amino aza-3 bicyclo [3,3,0] octane en continu, sans formation d'aucun intermédiaire toxique, mais il permet aussi l'obtention de l'hydrazine avec un coût peu élevé.

En effet, la synthèse classique de Raschig nécessite en général un grand excès d'amine, ce qui constitue un inconvénient considérable quand les amines utilisées comme matière première pour la préparation des hydrazines correspondantes ont un coût très élevé. C'est le cas d'aza-3 bicyclo [3,3,0] octane.

Le procédé de la présente invention (réaction en milieu biphasique, géométrie des réacteurs) permet de limiter cet excès d'aza-3 bicyclo [3,3,0] octane à moins de 5 fois par rapport à la quantité de monochloramine. De plus, grâce à la récupération et au recyclage d'aza-3 bicyclo [3,3,0] octane qui n'a pas réagi, il permet d'obtenir le N-amino aza-3 bicyclo [3,3,0] octane avec un coût très peu élevé par rapport aux autres procédés connus. L'isolement de l'aza-3 bicyclo [3,3,0] octane sous forme d'une solution aqueuse à relativement basse temperature constitue aussi une autre grande originalité ainsi qu'un avantage économique considerable du procédé de l'invention. Il est connu que l'aza-3 bicyclo [3,3,0] octane est un produit thermodégradable qui bout sous pression atmosphérique à seulement 184°C. Le procédé de la présente invention permet l'isolement de l'aza-3 bicyclo [3,3,0] octane sous forme d'hétéroazéotrope à une température moins élevée.

Un autre avantage du procédé résulte de la démixtion facile du N-amino aza-3 bicyclo [3,3,0] octane sous forme de solution concentrée (92% en N-amino aza-3 bicyclo[3,3,0] octane) par simple addition de soude au milieu réactionnel préalablement débarrassé d'ammoniaque et d'aza-3 bicyclo [3,3,0] octane.

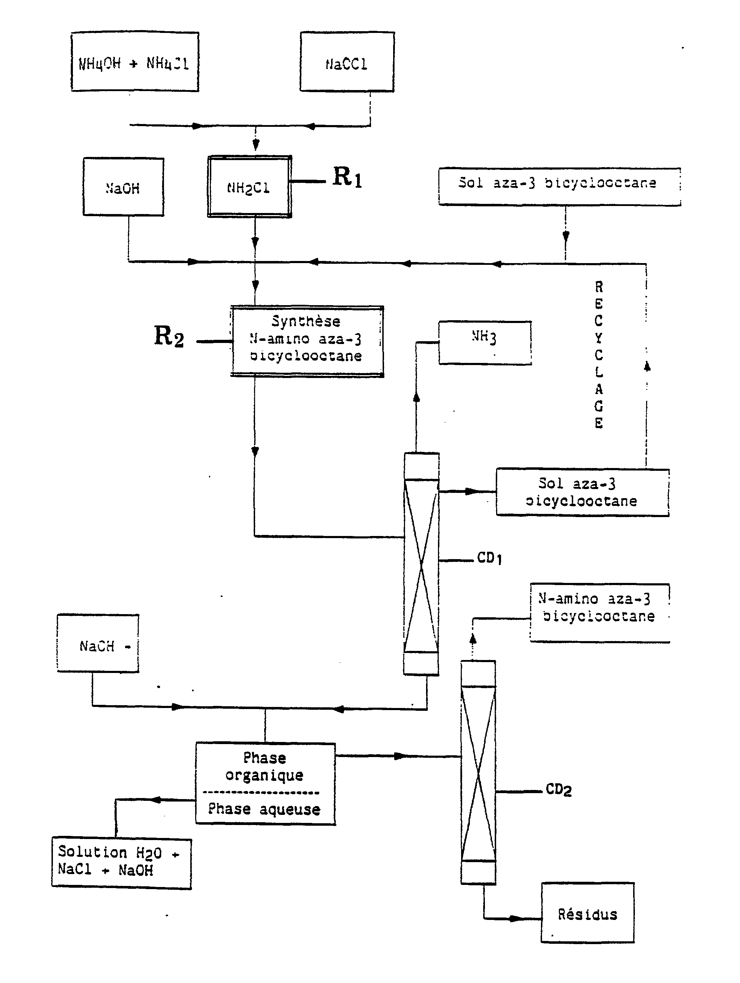

Ci-après est donnée à titre non limitatif une description détaillée de la mise en oeuvre du procédé de l'invention, procédé dont le schéma de principe est représenté à la figure 1.

EXEMPLE 1:

**Préparation de N-amino aza-3 bicyclo [3,3,0] octane**

Toutes les quantités indiquées correspondent à une unité en régime et sont rapportées à un litre d'hypochlorite injecté.

Un litre d'une solution d'hypochlorite de sodium titrant 48° chlorométriques et un litre d'une solution ayant une concentration en ammoniac de 3,60 mol.l$^{-1}$ et en chlorure d'ammonium de 2,37 mol.l$^{-1}$ sont introduits en continu dans un réacteur agité (R$_1$) à raison de 3,1 ml.mn$^{-1}$.

La température au sein du réacteur est maintenue entre -8° et -10°C, et le pH de la réaction est voisin de 10. A la sortie de R$_1$, on obtient une solution de monochloramine de titre supérieur à 1 mol.l$^{-1}$, ce qui correspond à un rendement proche de 100% par rapport à l'hypochlorite de sodium.

La synthèse de N-amino aza-3 bicyclo [3,3,0] octane est effectuée en milieu biphasique dans un réacteur cylindrique (R$_2$) agité énergiquement au moyen d'un agitateur coaxial à ailettes, de façon à maintenir le mélange émulsionné. La hauteur du réacteur est environ 25 cm et son volume 31,6 ml.

La solution de la chloramine obtenue ci-dessus (2 litres), la solution aqueuse de l'aza-3 bicyclo [3,3,0] octane (3,8 litres à 30%) et l'hydroxyde de sodium (0,5 litres à 6 mol.l$^{-1}$) sont introduits simultanément en continu dans le réacteur R$_2$ avec un débit adéquat pour avoir un rapport molaire aza-3 bicyclo [3,3,0] octane sur monochloramine environ 5,2 et un pH fixé à 13.4. La température de la réaction est maintenue à environ 50°C. Après 30 secondes de réaction, le mélange réactionnel est refroidi dans un échangeur à 16°C et redevient monophasique. On obtient un mélange de concentration voisine de 0,26 mol.l$^{-1}$ en hydrazine. Le mélange réactionnel subit ensuite une opération de dégazage pour éliminer l'ammoniac contenu dans la solution. La solution réactionnelle débarrassée de l'ammoniac est distillée à 98,4°C sous pression atmosphérique (colonne de distillation CD$_1$) pour éliminer l'aza-3 bicyclo [3,3,0] octane qui n'a pas réagi. L'amine est obtenue après distillation sous forme d'une solution aqueuse de composition d'environ 30% en amine. Cette solution est ensuite recyclée et utilisée immédiatement.

Après séparation d'aza-3 bicyclo [3,3,0] octane, la solution réactionnelle contenant l'hydrazine est traitée par addition d'hydroxyde de sodium solide, pour séparer le N-amino aza-3 bicyclo [3,3,0] octane dans une phase organique titrant près de 92% en hydrazine. Suivant les spécifications d'emploi, la solution concentrée de l'hydrazine peut-être ensuite utilisée directement ou distillée sous pression réduite (colonne de distillation CD$_2$).

Le rendement en hydrazine par rapport à l'aza-3 bicyclo [3,3,0] octane consommé est entre 88 et 92%.

**Revendications**

1. Procédé de synthèse en continu du N-amino aza-3 bicyclo [3,3,0] octane, caractérisé en ce que l'on fait réagir une solution d'hydroxyde d'ammonium et de chlorure d'ammonium avec une solution aqueuse d'hypochlorite de sodium à une température comprise entre-15°C et -7°C en milieu alcalin,
   – et ensuite, que l'on fait réagir la monochloramine ainsi formée avec l'aza-3 bicyclo [3,3,0] octane, en milieu biphasique dans un réacteur approprié muni d'un agitateur coaxial à ailettes, à une température comprise entre 30°C et 90°C et en milieu alcalin,
   – puis que l'on sépare du milieu réactionnel l'ammoniac et ensuite l'aza-3 bicyclo [3,3,0] octane qui n'a pas réagi par distillation pour le recycler,
   – puis que l'on isole par démixtion une solution concentrée de N-amino aza-3 bicyclo [3,3,0] octane par addition d'hydroxyde de sodium au milieu réactionnel, et que l'on purifie l'hydrazine ainsi obtenue, si on le désire, par distillation.

2. Procédé selon la revendication 1, caractérisé en ce que le rapport molaire aza-3 bicyclo [3,3,0] octane/monochloramine est supérieur à 4 et inférieur à 8.

3. Procédé selon la revendication 1 caractérisé en ce que la réaction de la monochloramine avec l'aza-3 bicyclo [3,3,0] octane soit réalisée à un pH compris entre 13 et 14.

4. Procédé selon la revendication 1 caractérisé en ce que l'excès d'aza-3 bicyclo [3,3,0] octane qui n'a pas réagi avec le monochloramine soit distillé à une température entre 90°C et 100°C sous pression atmosphérique, avant d'être recyclé.

## Claims

1. A process for continuous synthesis of N-amino-3-aza-bicyclo[3,3,0]octane characterized by reacting a solution of ammonium hydroxide and ammonium chloride with an aqueous solution of sodium hypochlorite at a temperature between −15°C and −7°C in an alkaline medium,
— then reacting the thus formed monochloroamine with 3-aza-bicyclo[3,3,0]octane in a two-phase medium in a suitable reaction vessel equipped with a blade agitator at a temperature between 30°C and 90°C in an alkaline medium,
— then separating by distillation the ammonia and subsequent the non-reacted 3-aza-bicyclo[3,3,0]octane from the reaction medium for recycling,
— then isolating under demixing a concentrated solution of N-amino-3-aza-bicyclo[3,3,0]octane by addition of sodium hydroxide to the reaction medium and, if desired, purifying the thus formed hydrazine by distillation.

2. The process according to claim 1, characterized in that the mole ratio of 3-aza-bicyclo[3,3,0]octane/monochloroamine is more than 4 and less than 8.

3. The process according to claim 1, characterized by reacting the monochloroamine and the 3-aza-bicyclo[3,3,0]octane at a pH between 13 and 14.

4. The process according to claim 1, characterized by distilling the excess of non-reacted 3-aza-bicyclo[3,3,0]octane and the monochloroamine at a temperature between 90°C and 100°C under atmospheric pressure prior to recycling.

## Patentansprüche

1. Verfahren zur kontinuierlichen Synthese von N-Amino-3-azabicyclo[3,3,0]octan, dadurch gekennzeichnet, daß man im alkalischen Medium eine Lösung aus Ammoniumhydroxid und Ammoniumchlorid mit einer wäßrigen Natriumhypochloritlösung bei einer Temperatur zwischen −15°C und −7°C umsetzt,
— dann im alkalischen Medium das in dieser Weise gebildete Monochloramin mit 3-Aza-bicyclo[3,3,0]octan im Zweiphasenmedium in einem geeigneten, mit einem Flügelrührer ausgerüsteten Reaktionsgefäß bei einer Temperatur zwischen 30°C und 90°C umsetzt,
— dann das Ammoniak und anschließend das 3-Aza-bicyclo[3,3,0]octan, das nicht umgesetzt worden ist, durch Destillation aus dem Reaktionsmedium zur Rückführung abtrennt,

— dann durch Zugabe von Natriumhydroxid zum Reaktionsmedium unter Entmischung eine konzentrierte Lösung aus N-Amino-3-aza-bicyclo[3,3,0]octan isoliert und, falls erwünscht, das in dieser Weise erhaltene Hydrazin durch Destillation reinigt.

2. Verfahren nach Anspruch 1, dadurch gekennzeichnet, daß das Molverhältnis von 3-Aza-bicyclo[3,3,0]octan/Monochloramin mehr als 4 und weniger als 8 beträgt.

3. Verfahren nach Anspruch 1, dadurch gekennzeichnet, daß man die Umsetzung des Monochloramins mit dem 3-Aza-bicyclo[3,3,0]octan bei einem pH-Wert zwischen 13 und 14 durchführt.

4. Verfahren nach Anspruch 1, dadurch gekennzeichnet, daß man den Überschuß an 3-Aza-bicyclo[3,3,0]octan, das nicht umgesetzt worden ist, mit dem Monochloramin vor der Rückführung bei einer Temperatur zwischen 90°C und 100°C unter Atmosphärendruck abdestilliert.